# EUROPEAN PATENT APPLICATION

(11) **EP 4 163 375 A1**
(43) Date of publication of application: **12.04.2023**
(21) Application number: 20928296.1
(22) Date of filing: 30.03.2020
(51) Int. Cl.: C12N 15/12, C12N 15/63, A61K 48/00, A61P 27/02, A61P 25/00

(54) **EXPRESSION VECTOR OF HUMAN NUCLEAR FACTOR E2-RELATED FACTOR 2 AND APPLICATION OF EXPRESSION VECTOR**

(71) Applicant: Wuhan Neurophth Biotechnology Limited Company, Wuhan, Hubei 430060 (CN)
(72) Inventor: LIU, Ting, Wuhan, Hubei 430060 (CN); LI, Bin, Wuhan, Hubei 430060 (CN)
(74) Representative: Cooley (UK) LLP
(86) International application number: PCT/CN2020/082162
(87) International publication number: WO 2021/195876

(57) **Abstract**

Provided are a nucleic acid encoding a human nuclear factor E2-related factor 2 (Nrf2) protein and a nucleotide sequence of the nucleic acid. Also provided are a recombinant expression vector containing the nucleic acid and a transformant introducing the nucleic acid into a host.

## Description

### Technical Field

The present invention relates to the field of biological preparations, and in particular to an expression vector for a human nuclear factor E2-related factor 2 and application thereof.

### Background

The optic nerve originates from the ganglion cell layer of the retina and is a part of the central nervous system, wherein the visual information obtained by the retina is transmitted to the brain through the optic nerve. There are many diseases related to optic nerve injury, including glaucoma, ischemic optic neuropathy, Leber's hereditary optic neuropathy (LHON), Autosomal dominant optic atrophy (DOA) and the like. The damage to ganglion cells and their axons is irreversible and is the main cause of vision loss and even blindness in patients. There are currently no effective drugs on the market for optic nerve injury. Drug research for optic nerve protection mainly focuses on neurotrophic factor drugs, such as BDNF, CNTF, etc., whose mechanism of action is to promote the growth of neurons and repair the damaged optic nerve, but the therapeutic effect is not ideal.

Therefore, there is an urgent need in the art to develop a method for treating optic nerve related diseases such as optic nerve injury with good therapeutic effect.

### Summary

A first object of the present invention is to provide a method for treating optic nerve related diseases such as optic nerve injury with good therapeutic effect.

A second object of the present invention is to, aiming at the deficiencies of the prior art, provide a nucleotide sequence encoding a human nuclear factor E2-related factor 2 (Nrf2) protein, which is capable of long-acting treatment of ophthalmic diseases;
A third object of the present invention is to provide an adeno-associated virus vector encoding the human nuclear factor E2-related factor 2 (Nrf2) protein;
A fourth object of the present invention is to provide the use of any of the nucleotide, adeno-associated virus vector, and plasmid in the treatment of acute or chronic optic nerve related diseases; wherein preferably, the diseases are glaucoma, DOA, LHON, ischemic optic neuropathy and the like.
A fifth object of the present invention is to provide a pharmaceutical preparation comprising the vector and a pharmaceutically acceptable carrier or excipient;
A sixth object of the present invention is to provide a delivery method for the pharmaceutical preparation, wherein the pharmaceutical preparation is injected intraocularly, and more preferably, the intraocular injection is intravitreal injection.

A first aspect of the present invention provides a nucleotide sequence encoding a human nuclear factor E2-related factor 2 (Nrf2) protein, wherein the nucleotide sequence is selected from the group consisting of:
(a) the nucleotide sequence as set forth in SEQ ID NO.: 1; and
(b) the nucleotide sequence having ≥95%, preferably ≥98%, and more preferably ≥99% identity with the nucleotide sequence as set forth in SEQ ID NO.: 1.

In another preferred embodiment, the nucleotide sequence includes a DNA sequence, a cDNA sequence, or an mRNA sequence.

In another preferred embodiment, the nucleotide sequence includes a single-stranded sequence and a double-stranded sequence.

In another preferred embodiment, the nucleotide sequence includes a nucleotide sequence that is completely complementary to SEQ ID NO.: 1.

The invention provides a vector comprising the nucleotide sequence as described in the first aspect of the present invention.

In another preferred embodiment, the vector comprises one or more promoters operably linked to the nucleic acid sequence, enhancer, transcription termination signal, polyadenylation sequence, origin of replication, selectable marker, nucleic acid restriction site, and/or homologous recombination site.

In another preferred embodiment, the promoter is selected from the group consisting of SYN, CMV, CAG, or a combination thereof.

In another preferred embodiment, the vector is selected from the group consisting of a plasmid and a viral vector.

In another preferred embodiment, the vector is selected from the group consisting of lentiviral vector, adenoviral vector, adeno-associated viral vector, or a combination thereof, and preferably, the vector is an AAV vector.

In another preferred embodiment, the serotype of the AAV vector is selected from the group consisting of AAV2, AAV5, AAV7, AAV8, or a combination thereof.

In another preferred embodiment, the vector comprises a DNA virus and a retrovirus vector.

In another preferred embodiment, the vector is an AAV vector comprising or inserted with the nucleotide sequence of claim 1; and preferably an AAV vector plasmid pAAV, pAAV-MCS, pAAV-MCS2, or pAAV-2Aneo.

In another preferred embodiment, the vector is used for expressing a recombinant human nuclear factor E2-related factor 2 (Nrf2) protein.

A third aspect of the present invention provides a host cell comprising the vector as described in the second aspect of the present invention, or having the exogenous nucleotide sequence as described in the first aspect of the present invention integrated into its chromosome.

In another preferred embodiment, the host cell is a mammalian cell, wherein the mammals include human and non-human mammals.

In another preferred embodiment, the host cell is selected from the group consisting of an HEK293 cell, an HEK293T cell, an HEK293T-17 cell, a photoreceptor cell (including a cone cell and/or a rod cell), other visual cells (such as a bipolar cell), a (optic) nerve cell, or a combination thereof.

In another preferred embodiment, the host cell is selected from the group consisting of a rod cell, a cone cell, an ON-bipolar cell, an OFF-bipolar cell, a horizontal cell, a ganglion cell, an amacrine cell, or a combination thereof, and preferably, the host cell is a (retinal) ganglion cell.

A fourth aspect of the present invention provides the use of the vector as described in the second aspect of the present invention in preparing a preparation or composition for treating optic nerve related diseases.

In another preferred embodiment, the preparation or composition is also used for one or more items selected from the group consisting of:
(a) activating anti-inflammatory or antioxidant responses of cells;
(b) increasing the survival rate of ganglion cells;
(c) preventing or delaying apoptosis of ganglion cells;
(d) causing stable high expression of Nrf2 gene in retinal ganglion cells; and
(e) scavenging free radicals from diseased cells.

In another preferred embodiment, the optic nerve related diseases include optic nerve injury.

In another preferred embodiment, the optic nerve related diseases include acute optic nerve injury and chronic optic nerve injury.

In another preferred embodiment, the optic nerve related disease is selected from the group consisting of glaucoma, autosomal dominant optic atrophy (DOA), Leber's hereditary optic neuropathy (LHON), ischemic optic neuropathy, or a combination thereof.

A fifth aspect of the present invention provides a pharmaceutical preparation comprising (a) the vector as described in the second aspect of the present invention, and (b) a pharmaceutically acceptable carrier or excipient.

In another preferred embodiment, the dosage form of the pharmaceutical preparation is selected from the group consisting of lyophilized preparation, liquid preparation, or a combination thereof.

In another preferred embodiment, the vector is selected from the group consisting of lentiviral vector, adenoviral vector, adeno-associated viral vector, or a combination thereof, and preferably, the vector is an AAV vector.

In another preferred embodiment, the amount of the vector in the pharmaceutical preparation is 1 × 10⁹ - 1 × 10¹⁶ viruses/ml, and preferably 1 × 10¹⁰ - 1 × 10¹² viruses/ml.

In another preferred embodiment, the pharmaceutical preparation is used for treating optic nerve related diseases.

A sixth aspect of the present invention provides a treatment method comprising administering the vector as described in the second aspect of the present invention to a subject in need thereof.

In another preferred embodiment, the vector is selected from the group consisting of lentiviral vector, adenoviral vector, adeno-associated viral vector, or a combination thereof, and preferably, the vector is an AAV vector.

In another preferred embodiment, the vector is introduced into the eyes of the subject in need thereof.

In another preferred embodiment, the subject in need thereof includes human and non-human mammals.

In another preferred embodiment, the treatment method is a method for treating optic nerve related diseases.

In another preferred embodiment, the administration comprises intraocular injection.

In another preferred embodiment, the administration comprises intravitreal injection.

A seventh aspect of the present invention provides a method of preparing a recombinant human nuclear factor E2-related factor 2 (Nrf2) protein, comprising the following step: culturing the host cell as described in the third aspect of the present invention to obtain the recombinant human nuclear factor E2-related factor 2 (Nrf2) protein.

It should be understood that, within the scope of the present invention, the above technical features of the present invention and the technical features specifically described in the following (e.g., examples) can be combined with each other to form new or preferred technical solutions. For brevity, no repeated description is provided here.

### Brief Description of the Drawings

Figure 1a is a comparison of the expression levels of Nrf2 protein after mouse retina is infected with rAAV2/2-NRF2_native and rAAV2/2-NRF2_opt viruses;
Figure 1b is a comparison of the transcription levels of Nrf2 downstream target gene *nqol* after mouse retina is infected with rAAV2/2-NRF2_native and rAAV2/2-NRF2_opt viruses;
Figure 2a is the result of TUJ1 staining in the retina of mice in the normal group (20x);
Figure 2b is the result of TUJ1 staining in the retina of mice in the control group;
Figure 2c is the result of TUJ1 staining in the retinal stretched preparation of mice in the rAAV2/2-Nrf2_native group;
Figure 2d is the result of TUJ1 staining in the retinal stretched preparation of mice in the rAAV2/2-Nrf2_opt group;
Figure 2e is the statistical result of the retinal ganglion cell density of mice in each group;
Figure 3a is the result of Brn3a staining in the retina of mice in the normal group (20x);
Figure 3b is the result of Brn3a staining in the retina of mice in the model group;
Figure 3c is the result of Brn3a staining in the retina of mice in the model +CAG-Nrf2_opt group;
Figure 3d is the result of Brn3a staining in the retina of mice in the model +SYN-Nrf2_opt group;
Figure 3e is the statistical result of the retinal ganglion cell density of mice in each group;
Figure 4a is a fluorescent photograph of eyeball slice of mice in the model +CAG-Nrf2_opt group;
Figure 4b is a fluorescent photograph of eyeball slice of mice in the model +SYN-Nrf2_opt group;
Figure 5a is a photograph of the fundus of the rabbit in the control group;
Figure 5b is a photograph of the fundus of the rabbit in experimental group A;
Figure 5c is a photograph of the fundus of the rabbit in experimental group B;
Figure 6a is the H&E staining in the eyeball slice of the rabbit in the control group;
Figure 6b is the H&E staining in the eyeball slice of the rabbit in the experimental group A;
Figure 6c is the H&E staining in the eyeball slice of the rabbit in the experimental group B;
Figure 7 is the profiles of plasmids illustrating the start-stop sites of each element including enhancers/promoters, gene sequences, polyAs, virus packaging ITR sequences, and Figure 7a, 7b, 7c and 7d are profiles of pAAV-CMV-Nrf2-native, pAAV-CMV-Nrf2_opt, pAAV-CAG-Nrf2_opt, and pAAV-SYN-Nrf2_opt plasmids, respectively.

### Detailed Description of the Invention

Through extensive and in-depth research, the inventors have carried out targeted optimization and design for the coding sequence of the recombinant human nuclear factor E2-related factor 2 (Nrf2) protein, thereby obtaining a nucleotide sequence that is particularly suitable for efficient transcription and expression of Nrf2 protein in mammalian (such as human) cells, and constructing a recombinant expression vector for the recombinant human nuclear factor E2-related factor 2 (Nrf2) protein. The transcription efficiency and translation efficiency of the specially optimized Nrf2 coding sequence (SEQ ID NO.: 1) were significantly improved, and the expression amount of the recombinant human nuclear factor E2-related factor 2 (Nrf2) protein was increased by 5 times or more. On this basis, the inventors have completed the present invention.

### Terms

In order that the present disclosure may be more readily understood, certain terms are first defined. As used in this application, unless expressly stated otherwise herein, each of the following terms will have the meaning given below. Other definitions are set forth throughout the present application.

The term "about" can refer to a value or composition within an acceptable error range of a particular value or composition as determined by one of ordinary skill in the art, which will depend in part on how the value or composition is measured or determined. For example, as used herein, the expression "about 100" includes all values between 99 and 101 (e.g., 99.1, 99.2, 99.3, 99.4, etc.).

As used herein, the terms "containing" or "including (comprising)" can be open, semi-closed, and closed. In other words, the term also includes "consisting essentially of", or "consisting of".

Sequence identity is determined by comparing two aligned sequence along a predetermined comparison window (which can be 50%, 60%, 70%, 80%, 90%, 95% or 100% of the length of the reference nucleotide sequence or protein), and determining the number of positions where identical residues occur. Typically, this is expressed as a percentage. The measurement of sequence identity of a nucleotide sequences is a method well known to those skilled in the art.

As used herein, the terms "subject", "subject in need thereof" refer to any mammal or non-mammal. Mammals include, but are not limited to, human, vertebrates such as rodents, non-human primates, cattle, horses, dogs, cats, pigs, sheep, and goats.

The terms "human nuclear factor E2-related factor 2 (Nrf2) protein", "Nrf2 (protein)", "polypeptide", "polypeptide of the present invention" and "protein of the present invention" are used interchangeably.

### Adeno-associated viruses

Adeno-associated virus (AAVs), also known as adeno associated viruses, belongs to the genus Dependovirus of the family Parvoviridae, and is a class of single-stranded DNA-deficient viruses with the simplest structure discovered so far, and its replication needs the participation of a helper virus (normally an adenovirus). It encodes the cap and rep genes in two inverted terminal repeats (ITRs). ITRs play a decisive role in viral replication and packaging. The cap gene encodes the viral capsid protein, and the rep gene is involved in the replication and integration of the virus. AAVs can infect a variety of cells.

Recombinant adeno-associated virus vector (rAAV) is derived from non-pathogenic wild-type adeno-associated virus, and is regarded as one of the most promising gene transfer vectors and have been widely used in gene therapy and vaccine research worldwide due to their characteristics such as good safety, wide range of host cells (dividing and non-dividing cells), low immunogenicity, and long-time expression of foreign genes in vivo. After more than 10 years of research, the biological characteristics of recombinant adeno-associated virus have been deeply understood, and a lot of data have been accumulated especially regarding its effect of application in various cells, tissues and in vivo experiments. In medical research, rAAV is used in research of gene therapy for various diseases (including in vivo and in vitro experiments); and also as a distinctive gene transfer vector, it is also widely used in gene function research, disease model construction, and preparation of gene knockout mice, etc.

In a preferred embodiment of the present invention, the vector is a recombinant AAV vector. AAV are relatively small DNA viruses that can be integrated into the genomes of the cells they infect in a stable and site-specific manner. They can infect a wide range of cells without any effect on cell growth, morphology or differentiation, and they do not appear to be involved in human pathology. The AAV genome has been cloned, sequenced and characterized. AAV comprises approximately 4700 bases, and an inverted terminal repeat (ITR) region of about 145 bases at each end, which serves as the origin of replication for the virus. The rest of the genome is divided into two important regions with encapsidation functions: a left part of the genome comprising the rep gene involved in viral replication and viral gene expression; and a right part of the genome comprising the cap gene encoding the viral capsid protein.

AAV vectors can be prepared using standard methods in the art. Adeno-associated virus of any serotype is suitable. Methods for purifying vectors can be found, for example, in US Patent Nos. 6,566,118, 6,989,264, and 6,995,006, the disclosures of which are incorporated herein by reference in their entirety. The preparation of hybrid vectors is described, for example, in PCT Application No. PCT/US2005/027091, the disclosure of which is incorporated herein by reference in its entirety. The use of AAV-derived vectors for gene transfer in vitro and in vivo has been described (see, e.g., International Patent Application Publication Nos. WO91/18088 and WO93/09239; US Patent Nos. 4,797,368, 6,596,535 and 5,139,941, and European Patent No. 0,488,528, all of which are incorporated herein by reference in their entirety). These patent publications describe various AAV-derived constructs in which the rep and/or cap genes are deleted and replaced by the gene of interest, as well as the use of these constructs in transferring the gene of interest in vitro (into cultured cells) or in vivo (directly into organisms). Replication-deficient recombinant AAVs can be prepared by co-transfection of the following plasmid into a cell line infected with a human helper virus (e.g., adenovirus): a plasmid containing a nucleic acid sequence of interest flanked by two AAV inverted terminal repeats (ITRs), and a plasmid carrying the AAV encapsidation genes (rep and cap genes). The resulting AAV recombinants are then purified by standard techniques.

In some embodiments, the recombinant vector is encapsidated into virus particles (e.g., including, but not limited to, AAV virus particles of AAV1, AAV2, AAV3, AAV4, AAV5, AAV6, AAV7, AAV8, AAV9, AAV10, AAV11, AAV12, AAV13, AAV14, AAV15 and AAV16, AAV-DJ). Accordingly, the present disclosure includes recombinant virus particles (they are recombinant because of comprising recombinant polynucleotides) comprising any of the vectors described herein. Methods of producing such particles are known in the art and described in US Patent No. 6,596,535.

### Nucleic acid coding sequence

The technical problem to be solved by the present invention is to overcome the technical defects of low transfection efficiency of a nuclear factor E2-related factor 2 (Nrf2) protein and poor therapeutic effect in the prior art. The invention provides a nuclear factor E2-related factor 2 (Nrf2) protein and a preparation method and application thereof. It is found through research that the optimized Nrf2 gene sequence (SEQ ID NO.: 1) of the present invention makes Nrf2 protein expression more efficient, thereby more Nrf2 protein plays a physiological role in the patient's optic ganglion cells.

The nucleotide sequence of the nucleic acid encoding the human nuclear factor E2-related factor 2 (Nrf2) protein as described in the present invention is set forth in SEQ ID NO.: 1. The nucleic acid encoding the human nuclear factor E2-related factor 2 (Nrf2) protein has a full length of 1818 bp. In the present invention, the nucleic acid encoding the human nuclear factor E2-related factor 2 (Nrf2) protein is also referred to as Nrf2-optimized gene or Nrf2-optimized nucleic acid.

The polynucleotide of the present invention may be in the form of DNA or RNA. In another preferred embodiment, the nucleotide is DNA. DNA forms include cDNA, genomic DNA or artificially synthetic DNA. DNA can be single-stranded or double-stranded. DNA can be the coding or non-coding strand.

The full-length nucleotide sequence of the present invention or a fragment thereof can typically be obtained by methods such as PCR amplification, recombinant method or artificial synthesis. For the PCR amplification, primers can be designed according to the published relevant nucleotide sequences, especially the open reading frame sequences, and commercial cDNA libraries or cDNA libraries prepared by conventional methods known to those skilled in the art can be used as templates for amplification to obtain the relevant sequences. When the sequence is long, it is often necessary to perform two or more PCR amplifications, and then splice the amplified fragments of each round together in the correct order. At present, the DNA sequence encoding the polypeptide of the present invention (or a fragment thereof, or a derivative thereof) can be obtained entirely by chemical synthesis. This DNA sequence can then be introduced into various existing DNA molecules (or e.g., vectors) and cells known in the art.

The present invention also relates to a vector comprising the polynucleotide of the present invention, as well as a host cell produced by genetically engineering with the vector or polypeptide coding sequence of the present invention. The above polynucleotide, vector or host cell may be isolated.

As used herein, "isolated" refers to the separation of a substance from its original environment (in the case of a natural substance, the original environment is the natural environment). For example, a polynucleotide and polypeptide in natural state in a living cell are not isolated and purified, but the same polynucleotide or polypeptide is isolated and purified if it is separated from other substances that exist in natural state.

In a preferred embodiment of the present invention, the nucleotide sequence is set forth in SEQ ID NO.: 1.

Once the relevant sequence has been obtained, the recombinant method can be used to obtain the relevant sequence in bulk. This is usually done by cloning it into a vector, transferring the vector into a cell, and isolating the relevant sequence from the proliferated host cell by conventional methods.

In addition, the artificial synthesis method can also be used to synthesize the relevant sequence, especially when the fragment length is short. A fragment with a very long sequence can typically be obtained by synthesizing multiple small fragments followed by ligation.

A method of amplifying DNA/RNA using PCR technology is preferred for obtaining the gene of the present invention. Primers for PCR can be appropriately selected according to the sequence information of the present invention as disclosed herein, and can be synthesized by conventional methods. The amplified DNA/RNA fragments can be isolated and purified by conventional methods such as gel electrophoresis.

The present invention also relates to a vector comprising the polynucleotide of the present invention, as well as a host cell produced by genetically engineering with the vector or protein coding sequence of the present invention, and a method for expressing Nrf2 protein by recombinant techniques using the host cell.

Through conventional recombinant DNA techniques, the host cell (e.g., mammalian cell) expressing the Nrf2 protein of the present invention can be obtained by using the polynucleotide sequence of the present invention. Generally, it includes the following step: transducing the polynucleotide of the first aspect of the present invention or the vector of the second aspect of the present invention into the host cell.

Methods well known to those skilled in the art can be used to construct an expression vector containing a DNA sequence encoding the polypeptide of the present invention and appropriate transcriptional/translational control signals. These methods include in vitro recombinant DNA techniques, DNA synthesis techniques, in vivo recombinant techniques, and the like. The DNA sequence can be effectively linked to an appropriate promoter in the expression vector to direct mRNA synthesis. The expression vector also comprises a ribosome binding site for translation initiation and a transcription terminator.

In addition, the expression vector preferably contains one or more selectable marker genes to provide phenotypic traits for selection of transformed host cells, such as dihydrofolate reductase, neomycin resistance and green Fluorescent protein (GFP) for eukaryotic cell culture, or tetracycline or ampicillin resistance for E. coli.

A vector comprising an appropriate DNA sequence and an appropriate promoter or a control sequence described above can be used to transform an appropriate host cell so that the cell can express the polypeptide.

The host cell can be a prokaryotic cell, or a lower eukaryotic cell, or a higher eukaryotic cell, such as a mammalian cell (including human and non-human mammals). Representative examples include animal cells such as CHO, NS0, COS7, or 293 cells. In a preferred embodiment of the present invention, HEK cells, HEK293 cells, HEK293T cells, HEK293T-17 cells, photoreceptor cells (including cone cells and/or rod cells), other visual cells (such as bipolar cells), and nerve cells are selected as host cells. In another preferred embodiment, the host cells are selected from the group consisting of rod cells, cone cells, ON-bipolar cells, OFF-bipolar cells, horizontal cells, ganglion cells, amacrine cells, or a combination thereof. Preferably, the host cell is a (retinal) ganglion cell.

Transformation of the host cell with recombinant DNA can be performed by using conventional techniques well known to those skilled in the art. When the host is a prokaryotic organism such as E. coli, a competent cell that can uptake DNA can be harvested after exponential growth phase and treated with CaCh method, wherein the procedures used are well known in the art. Another method is to use MgCh. If desired, transformation can also be performed by electroporation. When the host is a eukaryotic organism, the following DNA transfection methods can be selected: calcium phosphate co-precipitation, conventional mechanical methods such as microinjection, electroporation, liposome packaging, etc.

The obtained transformant can be cultured by conventional methods to express the protein encoded by the gene of the present invention. The medium used in the culture can be selected from various conventional mediums depending on the host cell used. Culture is carried out under conditions suitable for growth of the host cell. After the host cell has grown to an appropriate cell density, the promoter of choice is induced by an appropriate method (e.g., temperature switching or chemical induction), and the cell is cultured for an additional period of time.

The polypeptide in the above methods can be expressed in the cell, or on the cell membrane, or secreted outside the cell. If desired, the protein can be isolated and purified by various separation methods using its physical, chemical and other properties. These methods are well known to those skilled in the art. Examples of these methods include, but are not limited to: conventional renaturation treatment, treatment with protein precipitants (salting-out method), centrifugation, osmotic disruption of the bacteria, ultratreatment, ultracentrifugation, molecular sieve chromatography (gel filtration), adsorption chromatography, ion exchange chromatography, high performance liquid chromatography (HPLC) and various other liquid chromatography techniques and a combination thereof.

### Sequence optimization

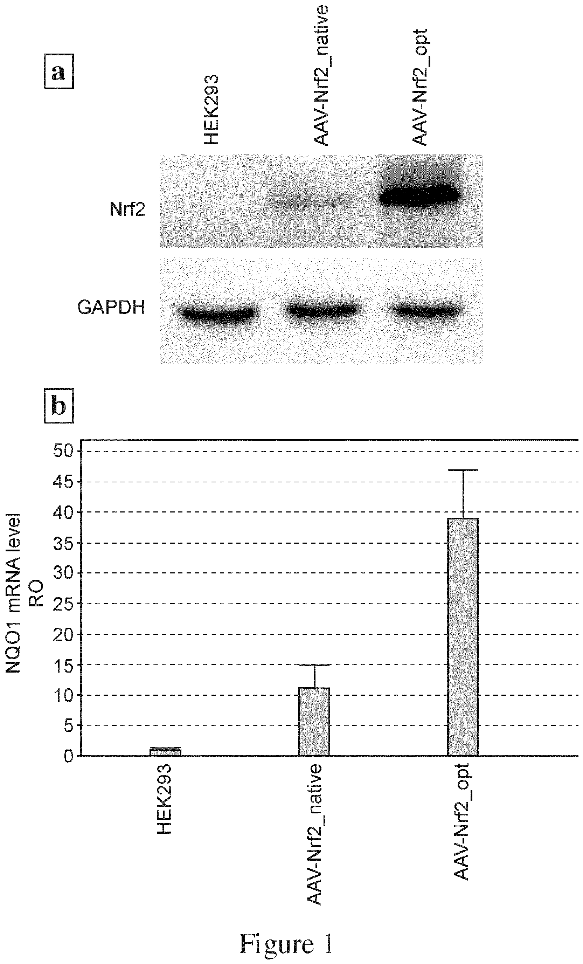

The present invention carries out Nrf2 nucleotide sequence optimization (as set forth in SEQ ID NO: 1, referred to as optimized Nrf2 gene/nucleic acid in the present invention), wherein this sequence is specially optimized, thus the transcription efficiency and translation efficiency are significantly improved as shown in Figure 1a, wherein the homology of optimized Nrf2 sequence and non-optimized Nrf2 sequence is 75%.

In the present invention, the sequence of the recombinant human nuclear factor E2-related factor 2 (Nrf2) protein optimized for nuclear codon bias is provided as set forth in SEQ ID NO.: 1.

As used herein, the "optimized Nrf2 coding sequence" and "optimized Nrf2 coding gene" both refer to a nucleotide sequence for encoding the recombinant human nuclear factor E2-related factor 2 (Nrf2) protein, and the amino acid sequence of the recombinant human nuclear factor E2-related factor 2 (Nrf2) protein is set forth in SEQ ID NO.: 3).

### Expression vector and host cell

The present invention also provides an expression vector for Nrf2 protein, which comprises the optimized Nrf2 coding sequence of the present invention.

With the sequence information provided, one skilled artisan can use available cloning techniques to produce a nucleic acid sequence or a vector suitable for transduction into a cell.

Preferably, the nucleic acid sequence encoding the Nrf2 protein is provided as a vector, preferably an expression vector. Preferably, it is provided as a gene therapy vector preferably suitable for transduction and expression in a retinal target cell. The vector can be viral or non-viral (e.g., a plasmid). Viral vectors include those derived from adenovirus, including mutated adeno-associated virus (AAV), retrovirus, lentivirus, herpes virus, vaccinia virus, MMLV, GaLV, simian immunodeficiency virus (SIV), HIV, poxvirus, and SV40. Preferably, the viral vector is replication defective, although it is envisaged that it may be replication deficient, replicable or conditionally replicable. The viral vector can generally remain in an extrachromosomal state without being integrated into the genome of the target retinal cell. A preferred viral vector for introducing the nucleic acid sequence encoding the Nrf2 protein into the retinal target cell is an AAV vector, for example, a self-complementary adeno-associated virus (scAAV). Selective targeting can be achieved using specific AAV serotypes (AAV serotype 2 to AAV serotype 12, AAV-DJ) or modified versions of any of these serotypes, including the AAV 4YF and AAV 7m8 vectors.

The viral vector can be modified to delete any non-essential sequence. For example, in AAV, the virus can be modified to delete all or part of the IX gene, Ela and/or Elb gene. For wild-type AAV, without the presence of helper viruses such as adenovirus, replication is very inefficient. For recombinant adeno-associated viruses, preferably, the replication and capsid genes are provided in trans (in the pRep/Cap plasmid), and only the 2ITR of the AAV genome is retained and packaged into the virion, meanwhile the adenoviral gene required being provided by adenovirus or another plasmid. Similar modifications can also be made to a lentiviral vector.

The viral vector has the ability to enter a cell. However, non-viral vectors such as a plasmid can be complexed with an agent to facilitate uptake of the viral vector by a target cell. Such agents include polycationic agents. Optionally, delivery systems such as a liposome-based delivery system can be used. The vector for use in the present invention is preferably suitable for use in vivo or in vitro, and preferably suitable for use in human. The plasmid is preferably the AAV vector plasmid pAAV, pAAV-MCS, pAAV-MCS2, or pAAV-2Aneo.

The vector will preferably comprise one or more regulatory sequences to direct expression of the nucleic acid sequence in the retinal target cell. Regulatory sequences can include a promoter, enhancer, transcription termination signal, polyadenylation sequence, origin of replication, nucleic acid restriction site, and homologous recombination site that operably linked to the nucleic acid sequence. The vector may also comprise a selectable marker, for example, to determine the expression of the vector in a growth system (e.g., a bacterial cell) or in a retinal target cell.

"Operably linked" means that the nucleic acid sequence is functionally related to the sequence to which it is operably linked, such that they are linked in such a way that they affect the expression or function of each other. For example, a nucleic acid sequence operably linked to a promoter will have an expression pattern affected by the promoter.

A promoter mediates the expression of the nucleic acid sequence to which it is linked. The promoter may be constitutive or may be inducible. A promoter can direct ubiquitous expression in an inner retinal cell, or neuron-specific expression. In the latter case, the promoter may direct cell-type-specific expression, for example, for an optic ganglion cell. Suitable promoters will be known to those skilled in the art. For example, a suitable promoter can be selected from the group consisting of: CAG, SYN, L7, thy-1, Restorin, Calbindin, Human CMV, GAD-67, Chicken Beta Actin, hSyn, Grm6, Grm6 Enhancer SV40 fusion protein. Targeting can be achieved using a cell-specific promoter, for example, Grm6-SV40 for selective targeting an optic nerve cell. The Grm6 promoter is a fusion of the 200 bp enhancer sequence of the Grm6 gene and the SV40 eukaryotic promoter, wherein the Grm6 gene encodes the optic nerve cell-specific metabotropic glutamate receptor mGluR6. Preferred sources of the Grm6 gene are mice and human. Ubiquitous expression can be achieved using a pan-neuronal promoter, examples of which are known and available in the art. One of such examples is CAG. The CAG promoter is a fusion of the CMV early enhancer and the chicken β-actin promoter.

An example of suitable promoters is the immediate early cytomegalovirus (CMV) promoter sequence. The promoter sequence is a strong constitutive promoter sequence that can drive high-level expression of any polynucleotide sequence operably linked thereto. Another example of suitable promoters is elongation growth factor-1α (EF-1α). However, other constitutive promoter sequences can also be used, including but not limited to the simian virus 40 (SV40) early promoter, mouse mammary tumor virus (MMTV), human immunodeficiency virus (HIV) long terminal repeat (LTR) promoter, MoMuLV promoter, avian leukemia virus promoter, Epstein-Barr virus immediate early promoter, Rous sarcoma virus promoter, and human gene promoters such as but not limited to the actin promoter, myosin promoter, heme promoter and creatine kinase promoter. Further, the present invention should not be limited to the use of constitutive promoters. Inducible promoters are also contemplated as part of the present invention. The use of an inducible promoter provides a molecular switch that can turn on expression of a polynucleotide sequence operably linked to an inducible promoter when such expression is desired, or turn off expression when the expression is not desired. Examples of inducible promoters include, but are not limited to the metallothionein promoter, glucocorticoid promoter, progesterone promoter, and tetracycline promoter.

In a preferred embodiment, the promoters of the present invention include, but are not limited to: CAG promoter, SYN1 promoter, and CMV promoter.

In a preferred embodiment, the sequence of the CAG promoter is set forth in SEQ ID NO.:4.

In a preferred embodiment, the sequence of the SYN1 promoter is set forth in SEQ ID NO.:5.

In a preferred embodiment, the sequence of the CMV promoter is set forth in SEQ ID NO.:6.

Many expression vectors can be used to express Nrf2 protein in a mammalian cell (preferably human, more preferably a human optic nerve cell or photoreceptor cell). In the present invention, adeno-associated virus is preferably used as an expression vector.

The present invention also provides a host cell for expressing Nrf2 protein. Preferably, the host cell is a mammalian cell (preferably human, more preferably a human optic nerve cell or photoreceptor cell) to increase the expression level of Nrf2 protein.

The present invention also provides a method of preparing a recombinant human nuclear factor E2-related factor 2 (Nrf2) protein, comprising the following step: culturing the above host cell to obtain the recombinant human nuclear factor E2-related factor 2 (Nrf2) protein.

The present invention also provides a treatment method comprising administering the vector to a subject in need thereof.

In another preferred embodiment, the vector is selected from the group consisting of lentiviral vector, adenoviral vector, adeno-associated viral vector, or a combination thereof. Preferably, the vector is an AAV vector.

In another preferred embodiment, the vector is introduced into the eyes of the subject in need thereof.

In another preferred embodiment, the subject in need thereof includes human and non-human mammals.

In another preferred embodiment, the treatment method is a method for treating optic nerve related diseases.

In another preferred embodiment, the optic nerve related disease is selected from the group consisting of glaucoma, autosomal dominant optic atrophy (DOA), Leber's hereditary optic neuropathy (LHON), ischemic optic neuropathy, or a combination thereof.

### Preparation and composition

The present invention provides a preparation or composition comprising (a) the vector of claim 2, and (b) a pharmaceutically acceptable carrier or excipient.

In another preferred embodiment, the dosage form of the pharmaceutical preparation is selected from the group consisting of lyophilized preparation, liquid preparation, or a combination thereof.

In another preferred embodiment, the vector is selected from the group consisting of lentiviral vector, adenoviral vector, adeno-associated viral vector, or a combination thereof, and preferably, the vector is an AAV vector.

In another preferred embodiment, the content of the vector in the pharmaceutical preparation is 1 × 10⁹ - 1 × 10¹⁶, preferably 1 × 10⁹ - 1 × 10¹² viruses/ml.

In another preferred embodiment, the pharmaceutical preparation is used for treating optic nerve related diseases.

In another preferred embodiment, the pharmaceutical preparation is used for one or more items selected from the group consisting of:
(a) activating anti-inflammatory or antioxidant responses of cells;
(b) increasing the survival rate of ganglion cells;
(c) preventing or delaying apoptosis of ganglion cells;
(d) causing stable high expression of Nrf2 gene in retinal ganglion cells; and
(e) scavenging free radicals from diseased cells.

The "active ingredient" in the pharmaceutical composition of the present invention refers to the vector of the present invention, for example, a viral vector (including an adeno-associated virus vector). The "active ingredient", preparation and/or composition of the present invention can be used to treat optic nerve related diseases. A "safe and effective amount" refers to an amount of the active ingredient sufficient to significantly improve the condition or symptoms without causing serious side effects. A "pharmaceutically acceptable carrier or excipient" refers to one or more compatible solid or liquid filler or gelling substances that are suitable for human use and that must be of sufficient purity and sufficient low toxicity. The "Compatibility" as used herein refers to that the components of the composition can be blended with the active ingredient of the present invention and with each other without significantly reducing the efficacy of the active ingredient.

The composition can be a liquid or solid, for example, powder, gel or paste. Preferably, the composition is liquid, preferably injectable liquid. Suitable excipients will be known to those skilled in the art.

In the present invention, the carrier can be administered to the eyes by subretinal or intravitreal administration. In either mode of administration, the carrier is preferably provided as an injectable liquid. Preferably, the injectable liquid is provided as a capsule or syringe.

Some examples of pharmaceutically acceptable carriers include cellulose and derivatives thereof (such as sodium carboxymethylcellulose, sodium ethylcellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agents (such as sodium lauryl sulfate), colorants, flavors, stabilizers, antioxidants, preservatives, pyrogen-free water, etc.

The composition may comprise physiologically acceptable sterile aqueous or non-aqueous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and suitable mixtures thereof.

The Nrf2-encoding nucleic acid or fusion nucleic acid provided by the present invention can produce Nrf2 protein in vitro or in vivo, wherein the protein or the preparation containing the protein can be used to prepare drugs for treating optic nerve related diseases.

The optimized nucleic acid encoding the human nuclear factor E2-related factor 2 (Nrf2) protein has higher expression level, thereby translating more Nrf2 protein. A medicament containing the optimized Nrf2 nucleic acid was injected into the vitreous cavity of rabbit eyes, wherein the medicament remained viable in the vitreous cavity, and the nucleic acid was transfected into optic nerve cells. The optimized Nrf2 nucleic acid encodes more Nrf2 protein than the prior art with higher transfection efficiency to treat optic nerve related diseases in a better way.

**Compared with the prior art, the present invention mainly has the following advantages:**
1. The encoding gene sequence of the recombinant human nuclear factor E2-related factor 2 (Nrf2) protein of the present invention has been specially optimized. Compared with the non-optimized DNA coding sequence of Nrf2, the transcription efficiency and translation efficiency are significantly improved, the codon usage is more efficient, and the Nrf2 protein expression level of the optimized sequence is significantly improved and the biological activity is high.
2. The optimized Nrf2 encoding gene (SEQ ID NO.: 1) of the present invention can treat optic nerve related diseases in a very effective way with good safety.
3. The present invention provides an adenovirus-related vector that can express Nrf2 protein in the human body, and the acute injury model in mice shows that the adenovirus-related vector can efficiently infect the retina and express a large amount of target genes, thereby activating cell anti-inflammatory and antioxidant responses and effectively increasing the survival rate of mouse RGCs. Therefore, it can be used for the treatment of acute or chronic optic nerve related diseases, such as optic nerve injury, glaucoma and the like.
4. The optimized sequence of the present invention has higher expression level and more stable expression in vivo, thereby improving the therapeutic effect of the drug.

The present invention will be further described below in conjunction with specific examples. It should be understood that these examples are only used to illustrate the present invention and not to limit the scope of the present invention. The experimental methods with no specific conditions indicated in the following examples usually follow conventional conditions, for example, conditions as described in Sambrook et al., molecular cloning: laboratory manual (New York: Cold Spring Harbor Laboratory Press, 1989), or conditions recommended by the manufacturer. Percentages and parts are by weight unless otherwise indicated.

Unless otherwise specified, the materials and reagents in the examples are all commercially available products.

### Example 1 Construction method of pAAV-Nrf2 vector

### 1.1 Vector construction

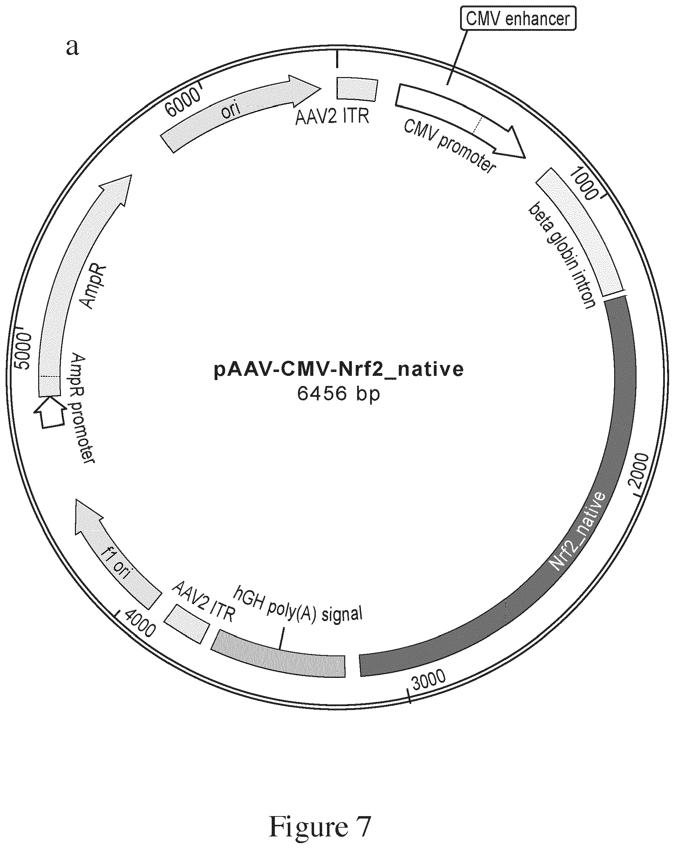

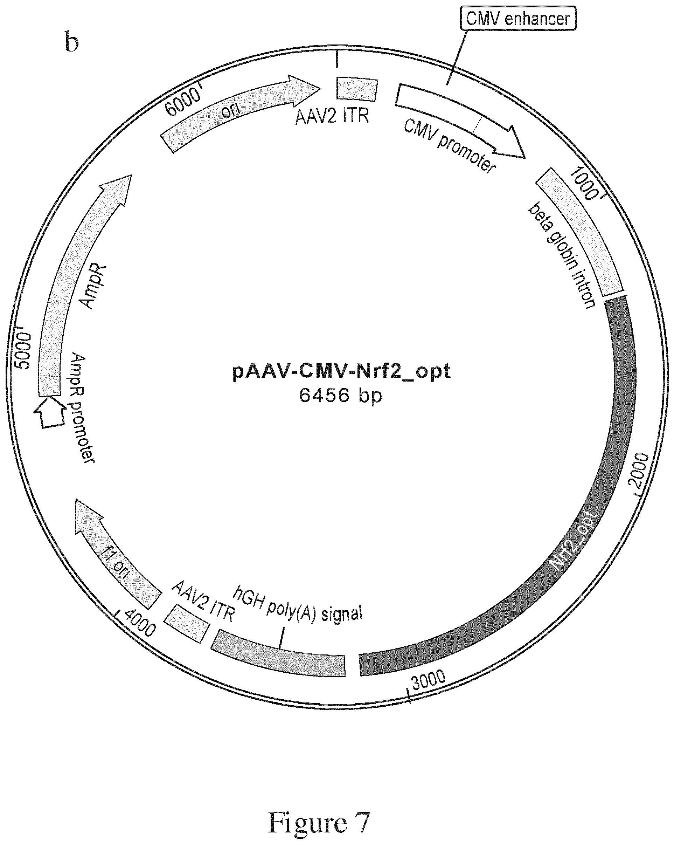

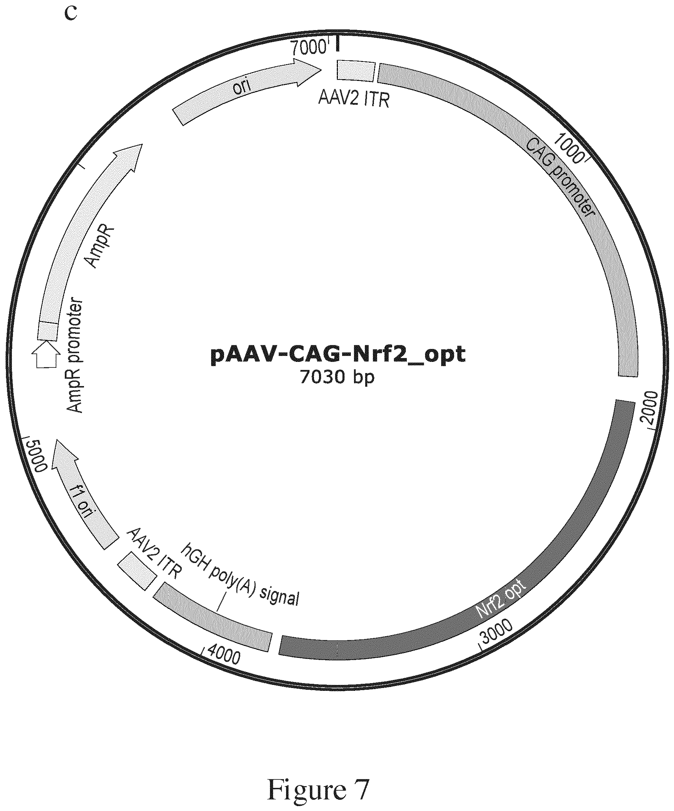

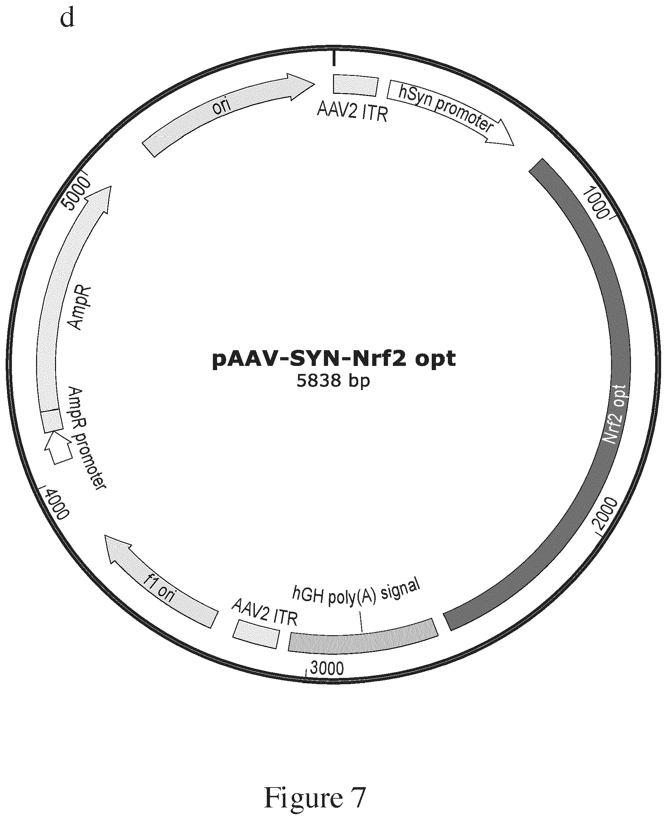

After obtaining the human Nrf2 nucleotide sequence (as set forth in SEQ ID NO.: 2), the Nrf2 nucleotide sequence optimization (as set forth in SEQ ID NO. 1) was carried out. Nucleic acid sequence optimization includes codon usage bias, elimination of secondary structures that are not conducive to expression (such as hairpin structures), changes in GC content, CpG dinucleotide content, mRNA secondary structure, cryptic splice sites, early polyadenylation sites, internal ribosome entry and binding sites, negative CpG islands, RNA instability regions, repeats (direct repeats, inverted repeats, etc.) and restriction sites that may affect cloning. The optimized sequence (SEQ ID NO: 1) was added with two restriction sites of Cla I and Xba I, or the product produced by PCR amplification with the primers designed with the new gene and the pAAV plasmid vector were subjected to Cla I and Xba I double-enzyme digestion, respectively, and then the digestion products were recovered and ligated with T4 DNA Ligase overnight, and then the ligation products were transformed into competent cells to obtain recombinant pAAV-Nrf2. A single clone was picked out for bacterial liquid sequencing, and the sequence obtained by sequencing was subjected to sequence alignment analysis to successfully obtain a recombinant adeno-associated virus plasmid pAAV-Nrf2 with the correct sequence, as shown in Figure 7, including enhancer/promoter, gene sequence, polyA, and viral packaging ITR sequence, wherein Figures 7a, 7b, 7c, and 7d are the profiles of pAAV-CMV-Nrf2_native, pAAV-CMV-Nrf2_opt, pAAV-CAG-Nrf2_opt, and pAAV-SYN-Nrf2_opt plasmids, respectively.

### 1.2 Coating of pAAV-Nrf2 recombinant adeno-associated virus

The pAdHelper, pAAV-r2c5, pAAV-Nrf2 plasmids were transfected into 293T cells (seeded in a 225cm² cell culture flask), and the cells were harvested after 48 hours. Cells were resuspended in PBS and freezed-thawed 3 times.

### 1.3 Purification and concentration of rAAV2/2-Nrf2 virus

The rAAV2/2-Nrf2 virus was isolated, concentrated and purified using three steps of chloroform treatment-PEG/NaCl precipitation-chloroform extraction. Total recovery = number of virus particles in final product/number of virus particles in starting material.

### 1.4 Fluorescence quantitative PCR method to detect the physical titer of rAAV2/2-Nrf2

Experimental materials: SYBR II (takara); target fragment primer (20uM); target plasmid for packaging virus (known concentration); virus to be tested; 8-tube PCR strip (Bio-red).

PCR reaction conditions: pre-denaturation: 95 °C for 10 min; cycle: 95 °C for 15 sec, and 60 °C for 1 min.

The titer of the genome was finally determined to be 1 × 10¹² vg/mL.

The preparation methods of other vectors are the same as above.

### Example 2 Comparison of expression levels and biological activities of rAAV2/2-Nrf2_native and rAAV2/2-Nrf2_opt in mice

### 2.1 Injection of recombinant adeno-associated virus into eye vitreous of mice

The mice were anesthetized by intraperitoneal injection with 5% chloral hydrate, and after the mice were anesthetized, the outer part of the eyes and the eyeballs of the mice were cleaned and disinfected. An insulin needle was used to make a hole under the corneoscleral margin of the mouse, and 1-2 µl of the recombinant adeno-associated virus preparation was intravitreally injected with a microsyringe, which consisted of the recombinant adeno-associated virus vector, and a pharmaceutically acceptable carrier or excipient. The mice were maintained in a standard environment with free diet, and the eye status of the mice was observed daily.

### 2.2 Comparison of expression levels and biological activities in retinas of mice

3 weeks after dosing, the mice were sacrificed by breaking spines with the eyeballs taken out, and the retinas were quickly peeled off on ice and stored under liquid nitrogen. After lysis of retinal tissue with RIPA buffer, the expression level of Nrf2 protein was detected by WB experiment. Figure 1a is a comparison of protein levels after rAAV2/2-Nrf2_native and rAAV2/2-Nrf2_opt viruses infected retinas of mice, wherein the protein expression level of the vector after sequence optimization is 5 times higher than that before optimization or more.

At the same time, RNA was extracted from retinas of mice, and then the transcription level of Nrf2-regulated downstream target gene *nqo*1 was detected by qPCR experiment. Figure 1b is a comparison of the transcription levels of Nrf2 downstream target gene after rAAV2/2-NRF_native and rAAV2/2-NRF_opt viruses infected retinas of mice, with the results showing that the transcription level of the downstream gene activated by the vector protein after sequence optimization is about 4 times higher than that before optimization. It shows that the vector after sequence optimization has stronger biological activity.

### Example 3 Protective effect on the optic nerve in the acute optic nerve injury model

### 3.1 Mouse model of acute optic nerve injury

Adult BL6/C57 mice were anesthetized by intraperitoneal injection of 5% chloral hydrate. The mice were fixed on an animal operating table, and the eye skin was disinfected with iodophor. The conjunctival tissue of the eyeball is cut along the sclera from the corneoscleral margin, and a bluntly backward separation was performed between the eyeball and the conjunctiva to expose the muscle vertebra. The separation fully exposed the optic nerve, and then the optic nerve was held for 10 s at 2 mm posterior to the eyeball with reverse self-locking elbow forceps with a width of 1 mm at the end of the forceps. Postoperative observation was performed and eye ointment was used to exclude the mice with fundus hemorrhage, lens damage and intraocular inflammation.

### 3.2 Experimental grouping and intravitreal injection

5 unmodeled mice were used as the normal group. Mice with successful modeling were divided into three groups for intravitreal injection. The control group was injected with rAAV2/2-mCherry virus (n=7), the experimental group A was injected with rAAV2/2-Nrf2_native virus (n=8), and the experimental group B was injected with rAAV2/2-Nrf2_opt virus (n=8).

### 3.3 Immunofluorescence staining of retinal ganglion cells and RGCs cell count

Mice were sacrificed by breaking spines, and the eyeballs were taken out and fixed in a fixative for 20 minutes, after which the retinas were peeled off, cut into 4 petaliform petals, and fixed in 4% paraformaldehyde at 4 °C overnight. The resulting materials were punched with 1% TritonX-100 for 2 hours, and blocked with the blocking solution for 1 hour. And then the resulting materials were incubated with TUJ1 antibody (ganglion cell-specific antibody) as the primary antibody at 4 °C overnight, and incubated with a secondary antibody at room temperature for 2 hours. The nuclei were DAPI-stained for 15 minutes before mounted.

Under the fluorescence microscope, 2 peripheral visual fields (3 mm from the optic disc) and 1 central visual field (1 mm from the optic disc) of the retinal temporal quadrant, superior quadrant, nasal quadrant, and inferior quadrant were selected for photographing and counting. 12 visual field images were collected for each retina, from which the average value was calculated, and then the number of retinal ganglion cells per unit area was calculated.

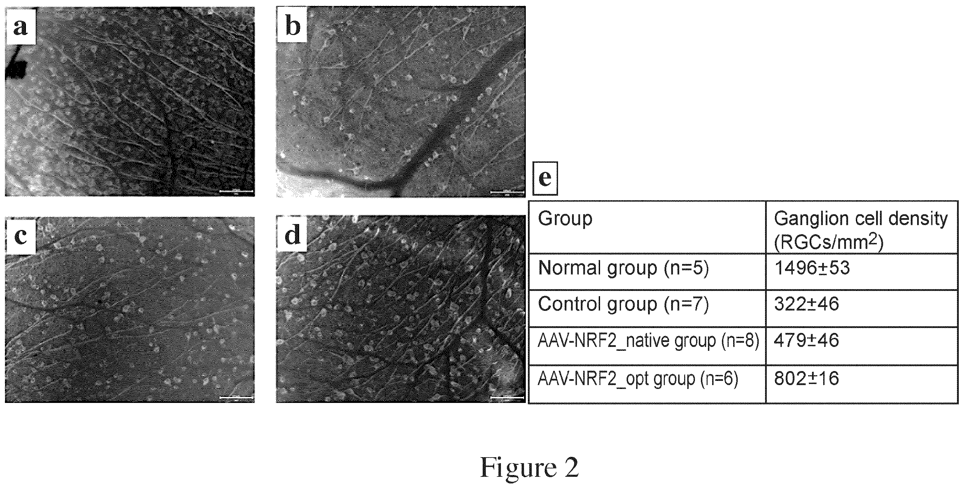

The results shown in Figure 2 shows that the density of surviving ganglion cells in the ONC modeling group (322±46 RGCs/mm², Figure 2b) was significantly lower than that in the normal group (1496±53 RGCs/mm², Figure 2a), with P<0.005, indicating a successful modeling. The densities of ganglion cells in the rAAV2/2-Nrf2_native drug group (479±46 RGCs/mm², Figure 2c) and the rAAV2/2-Nrf2_opt drug group (802±16 RGCs/mm², Figure 2d) were both significantly higher than that in the control group (322±46 RGCs/mm², Figure 2b), indicating a protective effect on retinal ganglion cells in modeling mice of drug groups. Herein, the number of surviving ganglion cells in mice of the rAAV2/2-Nrf2_opt drug group was significantly higher than that of the rAAV2/2-Nrf2_native drug group (P<0.05), indicating that the protective effect of the drug after sequence optimization on ganglion cells was better than that of the drug with natural sequence. Figure 2e shows the statistical results of the number of RGC cells per unit retinal area of mice in each group.

### Example 4 Protective effect on the optic nerve in glaucoma mouse model

### 4.1 Intravitreal injection of DBA/2J mice

7-month-old DBA/2J mice (intraocular pressure>18mmHg) were divided into three groups, wherein 6 mice were injected with PBS as the model group, and the other two groups were intravitreally injected with rAAV2/2-CAG-Nrf2_opt virus (n=7) and rAAV2/2-SYN-Nrf2_opt virus (n=7), respectively in single eyes. Five C57/BL6 mice of the same age were used as the normal group.

### 4.2 Intraocular pressure and fundus examinations for mice

Fundus and intraocular pressure examinations were performed every 3-5 days after surgery. There were no obvious abnormalities in the eyes of all mice, with no conjunctival hyperemia, secretion, endophthalmitis, or obvious changes in intraocular pressure.

### 4.3 Staining and counting of retinal ganglion cells

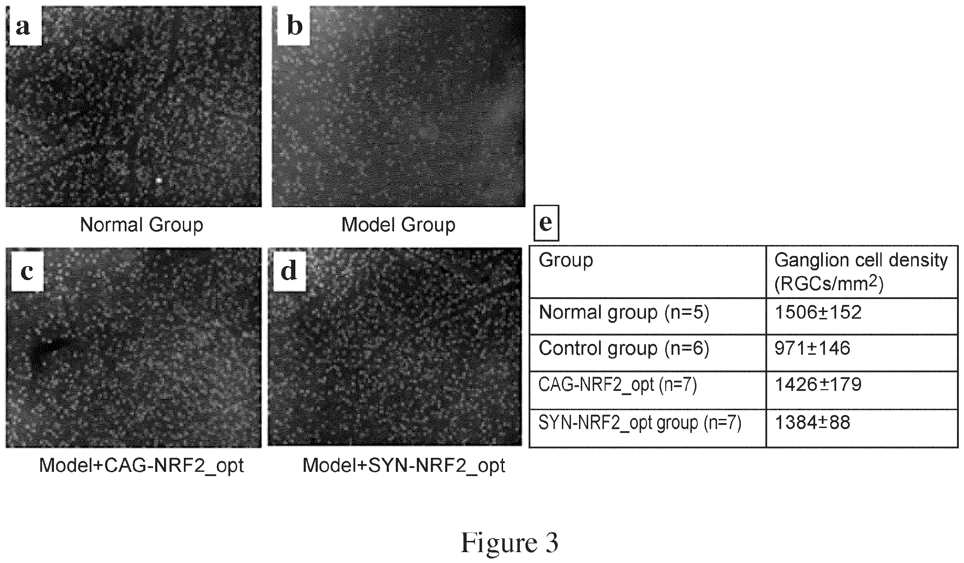

5 months after dosing, the mice were sacrificed by breaking spines, and the retinas were harvested for immunofluorescence staining and counting of ganglion cells. The results shown in Figure 3 shows that the density of surviving ganglion cells in mice of the model group (971±146 RGCs/mm², Figure 3b) was significantly lower than that of the normal group (1506±152 RGCs/mm², Figure 3a), with P<0.05. The densities of ganglion cells in the rAAV2/2-CAG-Nrf2_opt group (1426±179 RGCs/mm², Figure 3c) and the rAAV2/2-SYN-Nrf2_opt group (1384±88 RGCs/mm², Figure 3d) were significantly increased as compared with that in the model group (1506±152 RGCs/mm², Figure 3b) (P<0.05), indicating a protective effect on retinal ganglion cells in modeling mice of drug groups. However, there was no significant difference between the rAAV2/2-CAG-Nrf2_opt group and the rAAV2/2-SYN-Nrf2_opt group. Figure 3e shows the statistical results of the RGC cell counts per unit retinal area of mice in each group.

### 4.4 Retinal expression level

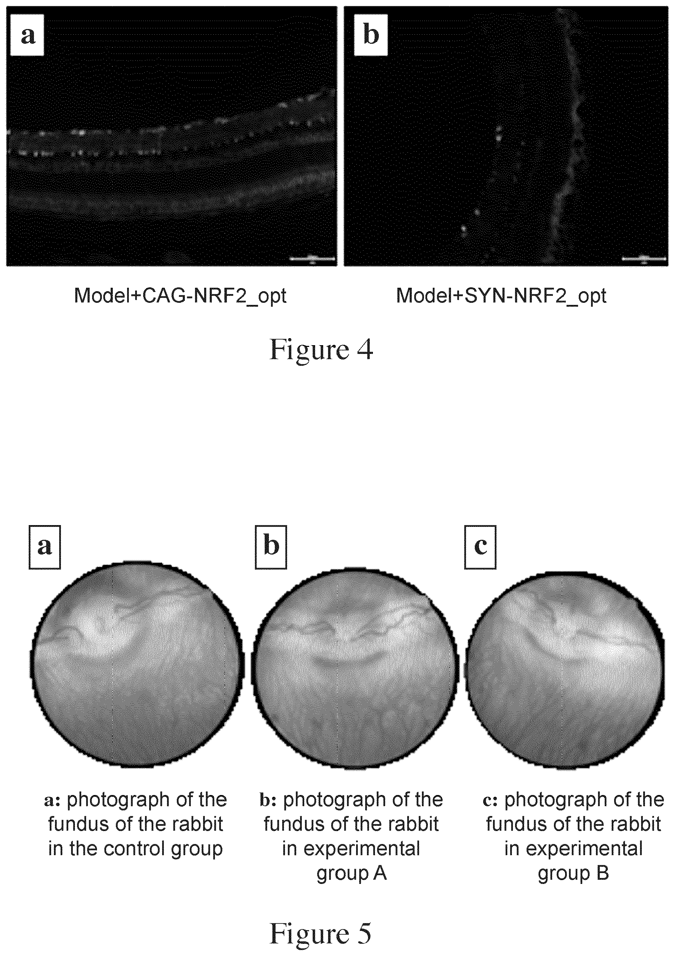

The eyeballs were taken for performing cryosection and immunofluorescence staining with Nrf2 antibody. The expression levels of rAAV2/2-CAG-Nrf2_opt and rAAV2/2-SYN-Nrf2_opt in retina were detected. The results shown in Figure 4 shows that after the retinas of mice were infected with rAAV2/2-CAG-Nrf2_opt, Nrf2 protein was strongly expressed not only in retinal ganglion cells, but also in bipolar cells and amacrine cells (Figure 4a). However, Nrf2 protein was mainly expressed in retinal ganglion cells following retinal infection with rAAV2/2-SYN-Nrf2_opt (Figure 4b), with higher specificity.

### Example 5 Expression of rAAV2/2-CAG-Nrf2_opt and rAAV2/2-SYN-Nrf2_opt in rabbit retina and safety detection

### 5.1 Intravitreal injection in rabbit eyes

27 New Zealand white rabbits were divided into 3 groups, including PBS group, experimental group A and experimental group B, and then 50ul 1 × 10¹² vg/mL of rAAV2/2-CAG-Nrf2_opt and rAAV2/2-SYN-Nrf2_opt were drawn respectively for intravitreal injection after puncturing the pars plana at 3 mm from the corneal limbus to enter the vitreous cavity.

### 5.2 Slit lamp, intraocular pressure, and fundus photography for examination

The three groups of rabbits were examined in terms of slit lamp and intraocular pressure at 1, 3, 7 and 30 days after surgery. All rabbits had no obvious abnormality, with no conjunctival hyperemia, secretion, endophthalmitis, or increase in intraocular pressure. The results of fundus photography of rabbits one month after surgery are shown in Figure 5. Compared with the fundus of rabbits in the control group (Figure 5a), there were no obvious complications or damage to the retinal blood vessels and optic nerves of rabbits in experimental group A (Figure 5b) and experimental group B (Figure 5c), indicating that the regular standard intravitreal injection will not cause obvious inflammatory response or other complications.

### 5.3 H&E staining of eyeball paraffin sections

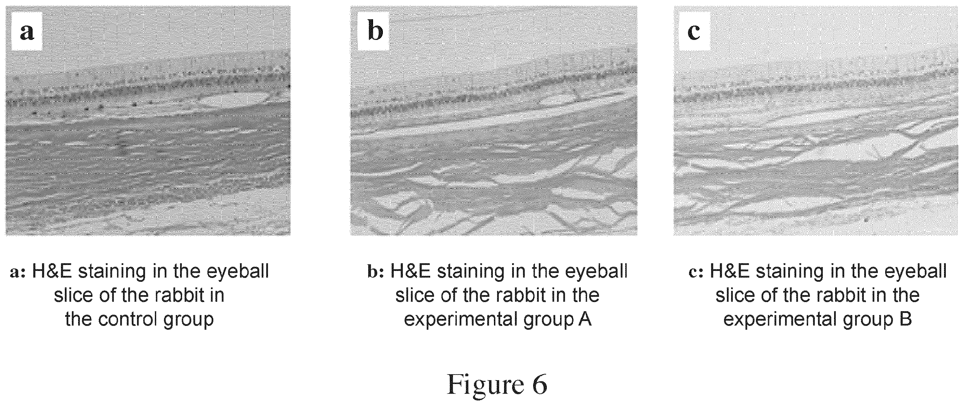

The results of H&E staining of rabbit eyeball sections showed that the numbers of retinal ganglion cells in experimental group A (Figure 6b), experimental group B (Figure 6c) and control group (Figure 6a) were basically the same, and the hierarchical structure was complete, indicating that the Nrf2_opt recombinant gene is safe, which causes no damage to the retina.

All documents mentioned in the present invention are incorporated by reference in this application as if each document were individually incorporated by reference. In addition, it should be understood that after reading the above teaching of the present invention, those skilled in the art can make various changes or modifications to the present invention, and these equivalent forms also fall within the scope defined by the appended claims of the present application.

## Claims

1. A nucleotide sequence encoding a human nuclear factor E2-related factor 2 (Nrf2) protein, wherein the nucleotide sequence is selected from the group consisting of:
(a) the nucleotide sequence as set forth in SEQ ID NO.: 1; and
(b) the nucleotide sequence having ≥95%, preferably ≥98%, and more preferably ≥99% identity with the nucleotide sequence as set forth in SEQ ID NO.: 1.

2. A vector, comprising the nucleotide sequence of claim 1.

3. A host cell, comprising the vector of claim 2, or having an exogenous nucleotide sequence of claim 1 integrated in its chromosome.

4. Use of the vector of claim 2 in preparing a preparation or composition for the treatment of optic nerve related diseases.

5. The use of claim 4, wherein the preparation or composition is also used for one or more items selected from the group consisting of:
(a) activating anti-inflammatory or antioxidant responses of cells;
(b) increasing the survival rate of ganglion cells;
(c) preventing or delaying apoptosis of ganglion cells;
(d) causing stable high expression of Nrf2 gene in retinal ganglion cells; and
(e) scavenging free radicals from diseased cells.

6. The use of claim 4, wherein the optic nerve related diseases include optic nerve injury.

7. The use of claim 4, wherein the optic nerve related diseases are selected from the group consisting of glaucoma, autosomal dominant optic atrophy (DOA), Leber's hereditary optic neuropathy (LHON), ischemic optic neuropathy, or a combination thereof.

8. A pharmaceutical preparation, comprising (a) the vector of claim 2, and (b) a pharmaceutically acceptable carrier or excipient.

9. The pharmaceutical preparation of claim 8, wherein the content of the vector in the pharmaceutical preparation is 1 × 10⁹ - 1 × 10¹⁶ viruses/ml, and preferably 1 × 10¹⁰ - 1 × 10¹² viruses/ml.

10. A method of preparing a recombinant human nuclear factor E2-related factor 2 (Nrf2) protein, comprising the step of culturing the host cell of claim 3 to obtain the recombinant human nuclear factor E2-related factor 2 (Nrf2) protein.
